# EUROPEAN PATENT APPLICATION

(11) **EP 1 925 660 A1**
(43) Date of publication of application: **28.05.2008**
(21) Application number: 08004456.3
(22) Date of filing: 25.05.2004
(51) Int. Cl.: C12M 1/00

(54) **Bioreactor for growing biological materials supported on a liquid surface**

(30) Priority: 30.05.2003 US 474479 P; 14.05.2004 US 845914
(62) Divisional of application: 04753293.2
(71) Applicant: Biolex, Inc., Pittsboro, NC 27312 (US)
(72) Inventor: Branson, Edward, R., Chapel Hill NC 27516 (US); Everett, Keith, Pittsboro NC 27312 (US); Hester, Bob, Morrisville NC 27560 (US); Vickers, Timothy, B., Chapel Hill NC 27517 (US)
(74) Representative: Jackson, Martin Peter

(57) **Abstract**

A bioreactor assembly (10) of the present invention for holding a media and supporting growth of a plurality of plants. The assembly includes a light source (13) and a container (11) having a flexible light transmissive wall structure and defining a reservoir. A major axis of the reservoir is substantially horizontal allowing the reservoir to be filled with media to a partial level and to define a relatively large surface area for support of the plants. The assembly may also include clamps (40) to secure and seal separate wall structure portions of the container together, and end caps to the wall portions, to define an aseptic environment. As another option, the clamps may define openings therethrough that allow passage of various devices for measuring and controlling bioreactor function such as a gas supply nozzle, a gas exit nozzle, an air temperature probe, a pH probe, a sampling drain, a gas composition probe and a media temperature probe.

## Description

### BACKGROUND OF THE INVENTION

### Field of the Invention

The present invention is related to the use of bioreactors for holding media and promoting the growth of biological materials, and in particular transparent bioreactors for growing biological materials requiring a light source, such as aquatic plants.

### Description of Related Art

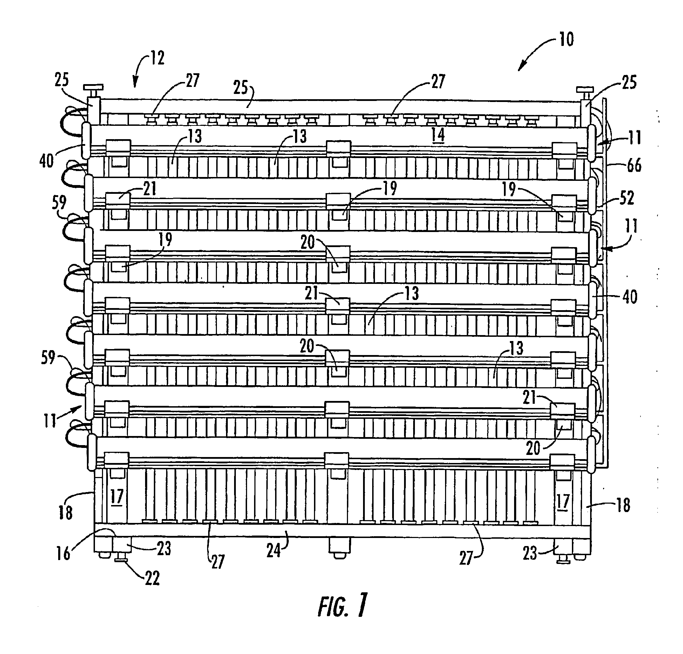

Photo-bioreactors are devices that allow photosynthetic microorganisms to grow in a controlled manner. U.S. Patent No. 5,846,816 to Forth ("Forth") discloses a biomass production apparatus including a transparent chamber 10 which has an inverted, triangular cross-section, as is shown in Figure 1 of Forth. Extending through the chamber is a first conduit 22 which has a plurality of perforations along its length to allow the introduction of gasses into the chamber. Also extending through the chamber are a pair of heat exchange conduits 26 connected to a supply of heat exchange medium.

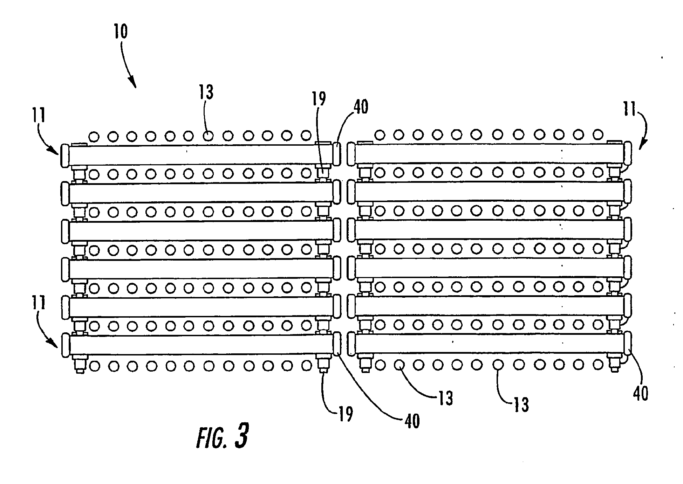

The passage of air entering through the conduit establishes a distinctive flow pattern that causes the liquid in the chamber to circulate up through a central region of the chamber, across the upper portion of the chamber below a cover 16, and down along the chamber sidewalls 20 back to the conduit, as is shown in Figure 3 of Forth. The cover includes two vents 28 through which the circulating gases exit the chamber. Ostensibly the passage of air and circulation of the liquid ensures that the biological matter suspended therein is exposed to light and also prevents the biological matter, such as algae, from adhering to the walls of the chamber.

Although the bioreactor disclosed by Forth promotes the growth of biological matter, it is generally not useful for applications requiring a sterile growth environment. The vents are open to external air which may include airborne contaminants. Such contaminants are especially troublesome for pharmacological applications wherein strict Food and Drug Administration guidelines for avoiding contamination must be met.

In addition, the constant circulation of the liquid required by Forth interferes with the growth of some types of biological matter. For instance, fully differentiated aquatic plants from the *lemnaceae* or "duckweed" family are fresh-water plants that grow best on the surface of the water. Such surface growing plants typically prefer relatively still water to support and promote optimal growth.

Therefore, it would be advantageous to have a photo-bioreactor system for promoting the growth of plant biological materials in a relatively sterile environment isolated from contaminants. It would be further advantageous if the system were capable of promoting growth of surface growing aquatic plants, such as the duckweed family of plants.

### BRIEF SUMMARY OF THE INVENTION

The above advantages are achieved and other needs addressed by a bioreactor assembly of the present invention for holding a media and supporting growth of a plurality of plants. The assembly includes a light source and a container having a light transmissive wall structure allowing light to pass therethrough and defining a reservoir filled with media and biological material. A major axis of the reservoir is substantially horizontal allowing the reservoir to be filled with media to a partial level and to define a relatively large surface area for support of plants and other biological materials that prefer such support. The assembly may also include clamps to secure and seal separate wall structure portions of the container together and end caps secured to the wall portions to maintain an aseptic environment. As another option, the clamps may define openings therethrough that allow passage of various devices for measuring and controlling bioreactor function such as a gas supply nozzle, a gas exit nozzle, an air temperature probe, a pH probe, a sampling drain, a gas composition probe and a media temperature probe.

In one embodiment, the present invention includes an assembly for holding a media and supporting growth of a plurality of plants. The assembly includes at least one light source and at least one container positioned adjacent the light source. A wall structure of the container has light transmissive properties to allow passage of light from the light source and in combination with other components defines a reservoir closed to the ambient environment. The reservoir has an elongate shape that defines a major axis generally extending in the direction of its longest dimension. In the assembly, the container is oriented so that the major axis of the reservoir is in a substantially horizontal plane with respect to gravity. In this manner, the reservoir, when partially filled with the media, creates a relatively large media surface on which the plants are supported.

In one aspect, the wall structure has an extruded shape with a constant cross-section. For instance, the wall structure may have a cylindrical, oval or rectangular cross-section. Preferred dimensions for the pipe wall structure range between 10 and 50 feet in length and between 2 and 12 inches in diameter. In cross-sections with a major axis, the major axis is preferably aligned with the substantially horizontal plane to further maximize the media surface area. For instance, two opposite corners of the rectangular cross-section could be positioned closer to the substantially horizontal plane than the remaining two corners.

In another aspect, a plurality of the containers may be used wherein the containers are arranged in a vertical stack with spacing between each of the containers. The vertical stack may be combined with electrically powered lights, such as light-emitting diodes or fluorescent lights as the light source. Light is supplied to the vertical stack by positioning the lights on both sides of the stack and potentially in the space between the containers.

Also included in the container may be one or more clamps for holding multiple portions of the wall structure together. The clamp may include one or more openings for the insertion of various sampling and control devices, such as a gas supply nozzle, a gas exit nozzle, an air temperature probe, a pH probe, a sampling drain, a gas composition probe and a media temperature probe extending into the reservoir through an opening defined in the clamp.

In another aspect, the clamp defines an opening that is sized and shaped to receive adjacent ends of the wall structure portions. For instance, the clamp may include a central band sized to extend around the clamp ends. Optionally, the clamp may have a pair of inwardly directed flanges spaced apart on opposite ends of the clamp wherein the flanges are configured to grip the ends of the wall structure portions. To facilitate gripping, the ends of the wall structure portions may flare outwardly to engage the inwardly directed flanges. Preferably, each of the clamps is constructed of an FDA approved composite material and includes a silicone seal to block contaminates from entering the container.

In an alternative embodiment, the container wall structure may define a closed reservoir having at least two spaced-apart portions each having a major axes. The major axes of the spaced apart portions lie in a common, substantially horizontal plane. In this manner, partially filling with media at one of the portions also partially fills the remaining portions and creates a media surface on which the plants are supported.

The present invention has many advantages. Overall, the bioreactor assembly allows the production of clinical and commercial scale quantities of biopharmaceuticals from genetically modified plants in a controlled, sterile and clean environment. For example, the use of containers defining reservoirs for partial filling with media provides a relatively large surface for the large-scale production of surface-borne biological materials, such as duck-weed plants. In addition, use of the clamps having seals to interconnect the various portions of the container wall structure and sealed openings for insertion of various measurement and supply devices ensures a clean and aseptic environment to promote the growth of the biological materials for medical uses. The clamping system also allows for easy assembly and disassembly of the containers for maintenance and modification. The measurement and supply devices ensure that the environment within the reservoir is tightly controlled to maximize growth and expression of the biological materials therein.

The present invention provides in a first aspect an assembly for holding a media and supporting growth of a biological material requiring light for proliferation, said assembly comprising: at least one light source; and at least one container positioned adjacent the light source, said container having a light transmissive wall structure defining an elongate, aseptic reservoir, said elongate reservoir having a major axis with a substantially horizontal orientation wherein said reservoir is capable of being partially filled with media so as to create a media surface on which the biological material is supported.

A second aspect differs from the first aspect in that the wall structure has a constant cross-section along said major axis.

A third aspect differs from the second aspect in that the cross-section is a circular cross-section.

A fourth aspect differs from the second aspect in that the wall structure is between 10 and 50 feet in length.

A fifth aspect differs from the fourth aspect in that the wall structure has a diameter from between 2 to 12 inches.

A sixth aspect differs from the second aspect in further comprising end caps secured to open ends of the wall structure.

A seventh aspect differs from the first aspect by additionally including a rack, with a plurality of said containers supported by said rack and arranged in a vertical stack with spacing therebetween.

An eighth aspect differs from the seventh aspect in that the light source includes a plurality of electrically powered lights positioned on opposite sides of the vertical stack.

A ninth aspect differs from the first aspect in that the wall structure has a constant oval-shaped cross-section along said major axis and wherein the oval shaped cross-section has a major axis extending in the substantially horizontal plane.

A tenth aspect differs from the first aspect in that the wall structure has a constant cross-section along said major axis and wherein the cross-section has a rectangular shape with two opposite corners of the rectangular shape structure positioned closer to the substantially horizontal plane than the remaining two opposite corners.

An eleventh aspect differs from the first aspect in that the container further includes at least one clamp and wherein the container wall is divided into at least two portions, wherein each of said portions has an open end and wherein the ends of the portions are adjacent and held together with a clamp.

A twelfth aspect differs from the eleventh aspect in that the clamp defines an opening sized and shaped to receive the adjacent ends of the wall structure portions.

A thirteenth aspect differs from the twelfth aspect in that the clamp includes a central band extending around the clamp opening and having a pair of inwardly directed flanges spaced apart on opposite ends of the clamp, said flanges configured to grip the ends of the wall structure portions.

A fourteenth aspect differs from the thirteenth aspect in that the ends of the wall structure portions flare outwards to engage the inwardly directed flanges.

A fifteenth aspect differs from the fourteenth aspect in that the clamp further includes a seal extending along the flanges between the flared ends of the walls structure portions and the inwardly directed flanges of the clamp.

A sixteenth aspect differs from the fifteenth aspect in that the clamp is constructed of an FDA approved composite material.

A seventeenth aspect differs from the fifteenth aspect in that the seal is an FDA approved elastomeric material.

An eighteenth aspect differs from the first aspect by further comprising at least one of a gas supply nozzle, a gas exit nozzle, an air temperature probe, a pH probe, a sampling drain, a gas composition probe and a media temperature probe extending into the reservoir through an opening defined by the container.

A nineteenth aspect provides an assembly for holding a media and supporting growth of a biological material requiring light for proliferation, said assembly comprising: a support rack; a plurality of elongate laterally extending containers carried by said support rack and arranged in a stack spaced apart vertically from one another, each container having a light transmissive wall structure defining an elongate, aseptic reservoir, said elongate reservoir having a major axis with a substantially horizontal orientation wherein the reservoir is capable of being partially filed with media so as to create a media surface on which the biological material is supported; and at least one light source carried by said support rack and mounted adjacent to said containers for illuminating the containers.

A twentieth aspect differs from the nineteenth aspect in that said support rack includes a plurality of upright support members, and upper and lower laterally extending support rails interconnecting said upright support members, and wherein said containers are mounted to said upright support members.

A twenty first aspect differs from the nineteenth aspect in that each of said containers comprises an elongate transparent tube of substantially uniform cross-section, and end caps closing opposite ends of said tube to define said elongate reservoir.

A twenty second aspect differs from the twenty first aspect in that each of said containers includes at least two elongate transparent tube sections mounted end-to-end, and a clamp interconnecting the adjoining end portions of the tube sections.

A twenty third aspect differs from the nineteenth aspect in that said at least one light source comprises a plurality of elongate fluorescent tubes mounted to said rack.

A twenty fourth aspect differs from the twenty third aspect in that the tubes extend vertically and are laterally spaced from one another.

A twenty fifth aspect differs from the twenty third aspect in that the tubes extend substantially horizontally and generally parallel to the containers.

A twenty sixth aspect provides a method of growing in a liquid media a biological material requiring light for proliferation, said method comprising: providing at least one light transmissive container defining a reservoir having a major axis with a substantially horizontal orientation; filling the reservoir with the liquid media until a partial fill level is reached so as to define a top surface of the media extending along a length of the reservoir ; adding the biological material to the reservoir and supporting the biological material on the top surface of the media; and exposing the container to a light source so as to promote growth of the biological material via photosynthesis.

A twenty seventh aspect differs from the twenty sixth aspect in that further comprising sealing the reservoir against contamination after filling the reservoir with liquid media and adding the biological material.

A twenty eight aspect differs from the twenty seventh aspect in that providing the light transmissive container includes clamping portions of the light transmissive container together using one or more clamps.

A twenty ninth aspect differs from the twenty eighth aspect by further comprising accessing the reservoir through an opening defined in the container.

A thirtieth aspect differs from the twenty ninth aspect in that accessing the reservoir includes inserting a gas supply nozzle through the clamp opening and supplying gas to the reservoir.

A thirty first aspect differs from the twenty ninth aspect in that accessing the reservoir includes inserting a temperature probe through the clamp opening and measuring a temperature within the reservoir.

A thirty second aspect differs from the twenty ninth aspect in that accessing the reservoir includes inserting a pH probe through the clamp opening and measuring a pH of the media within the reservoir.

A thirty third aspect differs from the twenty ninth aspect in that accessing the reservoir includes draining a sample through the clamp opening.

A thirty fourth aspect differs from the twenty sixth aspect in that filling the reservoir includes supplying the media through an opening defined in an end of the container.

A thirty fifth aspect differs from the twenty sixth aspect by further comprising draining the liquid media from the reservoir after filling the reservoir.

A thirty sixth aspect differs from the twenty sixth aspect by further comprising automatically measuring and controlling one of a temperature, a media pH, the media fill level, gas pressure and gas concentration.

A thirty seventh aspect differs from the twenty sixth aspect by further comprising supplying conditioned air around the container to control a temperate within the reservoir.

A thirty eighth aspect differs from the twenty sixth aspect by further comprising heating and circulating the media.

A thirty ninth aspect differs from the twenty sixth aspect by further comprising mounting a plurality of the containers in a stack spaced apart vertically from one another.

### BRIEF DESCRIPTION OF THE SEVERAL VIEWS OF THE DRAWING (S)

Having thus described the invention in generaltenns, reference will now be made to the accompanying drawings, which are not necessarily drawn to scale, and wherein:
Figure 1 is a side elevation view of a bioreactor assembly of one embodiment of the present invention;
Figure 2 is a front elevation view of the bioreactor assembly of Figure 1;
Figure 3 is an elevation view of a bioreactor assembly of another embodiment of the present invention;
Figure 4 is an elevation view of a bioreactor assembly of yet another embodiment of the present invention;
Figure 5 is a side elevation view of a bioreactor assembly of another embodiment of the present invention using relatively large diameter containers;
Figure 6 is a side elevation view of a bioreactor assembly of another embodiment of the present invention using oval shaped containers;
Figure 7 is a side elevation view of a bioreactor assembly of another embodiment of the present invention using angled, rectangular shaped containers;
Figure 8 is a side elevation view of an end of one of the containers shown in the bioreactor assembly of Figures 1 and 2;
Figure 9 is a plan view of an outer band portion of a clamp assembly of another embodiment of the present invention;
Figure 10 is a plan view of an inner sealing portion for combination with the outer band portion shown in Figure 9 to form the clamp assembly;
Figure 11 is a side elevation view of a bioreactor assembly of another embodiment of the present invention where a wall structure of the container has multiple portions held together by clamps; and
Figure 12 is a plan view of the bioreactor assembly of Figure 11.

### DETAILED DESCRIPTION OF THE INVENTION

The present invention now will be described more fully hereinafter with reference to the accompanying drawings, in which some, but not all embodiments of the invention are shown. Indeed, the invention may be embodied in many different forms and should not be construed as limited to the embodiments set forth herein; rather, these embodiments are provided so that this disclosure will satisfy applicable legal requirements. Like numbers refer to like elements throughout.

A bioreactor assembly **10** of one embodiment of the present invention is shown in Figure 1. Included in the bioreactor system are a plurality of substantially horizontally extending containers **11** supported by a rack **12** in a vertical stack adjacent a plurality of vertically extending lights **13.** Each of the containers **11** includes a light transmissive cylindrical wall structure **14** that defines a reservoir **15** for holding media at a partially full level so as to provide a surface for supporting duckweed, or other biological material, that requires light for growth.

The term "media" as used herein refers to any liquid, gel, partially liquid-partially solid, or otherwise flowable supply of compounds, chemicals or nutrients that are used to promote the growth, testing, modification or manipulation of the biological matter housed within the reservoir **15.** Media therefore, can be water alone, a combination of water with fertilizer, soil, an agar gel, mud or other combination of components, with or without water, as long as some type of flow and manipulation of the components can be induced using the devices described herein.

The term "biological materials" or "biological matter" as used herein describe any material that requires light and a supply of media in order to support proliferation or expression. Preferably, the biological materials are plants that require or thrive on liquid surfaces, such as plants within the duckweed family. Other preferred aquatic plants include Giant Salvinia, Kariba weed, Aquarium watermoss, Water Fern, Carolina mosquito fern, water hyacinth, jacinthe d'eau, Variable-leaf Pondweed, Waterthread Pondweed, Hydrilla, American Water-Plantain, Marsh Pennywort, and Creeping Rush. These plants and other biological material may be either wild plants, or transgenic plants for the production of vaccines, therapeutic proteins and peptides for human or animal use, neutraceuticals, small molecule pharmaceuticals, research and production reagents (growth factors and media additives for cell culture) or excipients for pharmaceuticals.

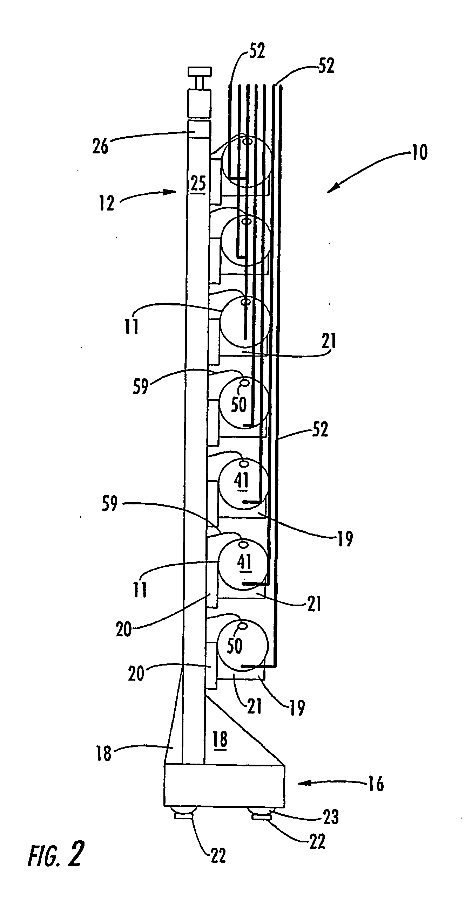

The rack **12** of the bioreactor assembly **10** includes a base **16** for supporting the remaining portions of the rack, as is shown in Figures 1 and 2. In particular, the base **16** includes multiple feet **22** resting on a floor or other supporting surface at the bottom of three leg members **23** positioned at the lateral ends and center of the rack. Resting on the leg members is a support rail **24** that extends horizontally and is substantially the length of the containers **11.**

Three vertical support members **17** are connected to and extend upwards from each lateral side and the center of the horizontal support rail **24.** Preferably, the vertical support members **17** are supported nearer a rear edge of the base **16** so as to provide additional stability for the containers **11** which are supported on cantilevered support members **19** that extend towards a front edge of the base.

Each of the cantilevered support members **19** is mounted to a front edge of the vertical support member at regularly spaced intervals. In addition, each of the cantilevered support members includes a mounting plate **20** attached to the vertical support member **17** on its rear surface and a retaining member **21** on its front surface. As is shown in Figure 2, the retaining member may define an arcuate upper surface congruently shaped and sized to match the outer surface of the container wall structure **14** so as to provide relatively snug support for the container.

In the embodiment illustrated in Figures 1 and 2, an additional pair of vertical support members **25** extend upwards from the horizontal support rail **24** of the base at the outermost ends of the horizontal support rail. A pair of gussets **18** reinforce the connection of the outermost ones of the vertical support members **25** to the horizontal support rail **24.** Each of the gussets has a triangular shape with one leg attached to the horizontal support rail **24** and the other leg attached to the vertical support members **25.** Because of the rearward positioning of the vertical support members, the base leg of the front one of the gussets **18** is longer than the base leg of the rear one of the gussets.

Supported at the top ends of each of the vertical support members **17, 25** is another horizontal support rail **26** that is equal in length and extends parallel to the bottom support rail **24.** Both the top and bottom horizontal support rails support a plurality of light mounts **27.** The light mounts are positioned in corresponding pairs extending along the rails **24, 26** at regular, spaced intervals. In this manner, each pair of light mounts can support a vertically extending one of the lights **13.**

The lights **13** are preferably artificial lights that are electrically powered. For instance, lighting can be supplied by light-emitting diodes, fluorescent lights, incandescent lights, sodium vapor lights, metal halide lights or various combinations of these, and other, types of lights. Alternatively, the artificial lights may also be aided by, or replaced with, direct and indirect sunlight. However, artificial lights are preferred due to their ease of control and positioning so that all of the duckweed, or other biological material, contained in the reservoir **15** is supplied a sufficient amount of light to promote growth. Supplying power to the various types of lights can be done via wiring, or other manner that is conventional in the art and therefore not described herein in additional detail.

As noted above, the lights **13** of the embodiment illustrated in Figure 1 have a vertical orientation (i.e., in the direction of gravity) which is perpendicular to the substantially horizontal orientation of the containers **11** held in the rack **12.** The vertically-oriented lights **13** are positioned on one side of the stack of containers **11** and are spaced in parallel at regular intervals along the wall structure **14** of the containers. In this manner each light provides illumination for an adjacent section of every one of the containers **11** from one end of each of the containers to the opposite end of the container.

Various different configurations of the lights **13** are possible depending upon different factors such as the intensity of the lighting need to support growth, the positions of the containers **11,** or the desired temperature of the media in the reservoir **15.** For instance, an alternative configuration for the lights **13** is shown in Figure 3. In this embodiment, the lights extend between the containers in a spaced, parallel arrangement. Notably, the lights in this arrangement may extend between the containers of several back-to-back vertical container stacks similar to the vertical stack shown in Figures 1 and 2.

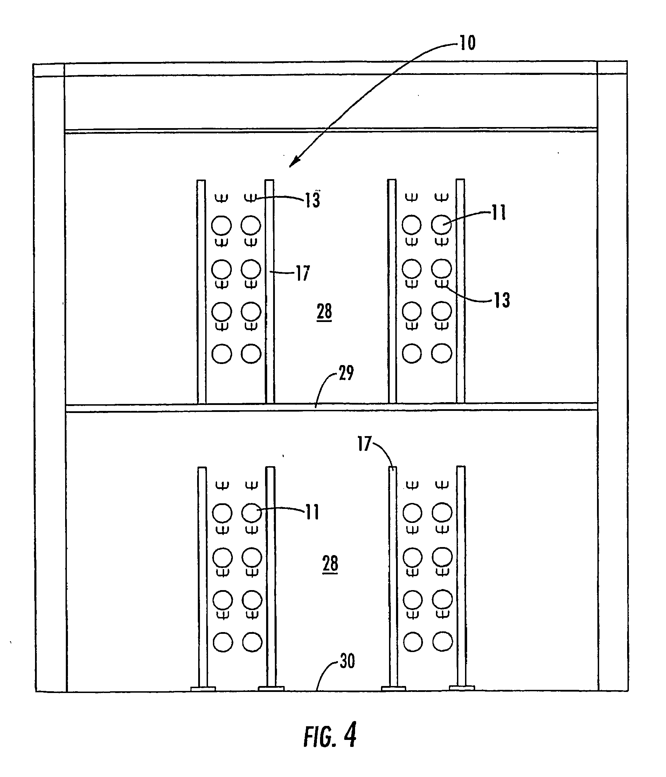

Another alternative configuration for the lights **13** is shown schematically in Figure 4, wherein the lights extend horizontally in pairs within the vertical spacing between pairs of the containers **11.** As a result, each of the lights extends in parallel along the length of a respective one of the containers **11.** Positioning above the container allows each pair of lights **13** to cast illumination downwards along the entire top length of the wall structure **14** of the container. Such an arrangement may be better suited for biological materials that reside mostly on the top surface of the media.

The containers **11** in the embodiment illustrated by Figure 4 are positioned in adjacent pairs of vertical stacks. The rack **12** in this embodiment includes vertical support members **17,** but does not necessarily require the support rails **24, 26** to support the lights **13,** unless needed for additional stability of the vertical support members. Extending inwards from the vertical support members are the cantilevered support members **19** (which are not shown in Figure 4 for clarity) which may be connected at their ends to the ends of the cantilevered support members of the adjacent vertical stack of containers **11.** Each pair of lights **13** is attached to the bottom of the retaining member **21** of the cantilevered support member above its respective one of the containers **11.**

The relative arrangement of the containers **11** in Figure 4 could also be accomplished by placing the front ends of two of the bioreactor assemblies illustrated in Figures 1 and 2 in an adjacent relationship. Such an arrangement would also combine the horizontally extending lights **13** of Figure 4 with the vertically extending lights of Figures 1 and 2. Advantageously, the side-by-side relationship allows for overlap between the lighting of the adjacent vertical stacks of containers **11.**

Another advantage of the side-by-side positioned vertical stacks of containers **11** shown in Figure 4 is that density of the containers is improved while at the same time allowing the containers and lights **13** to be easily accessed for service. For instance, each pair of vertical stacks could be spaced so as to provide a service aisle **28** between them. In addition, the density of the stacks is still low enough that conventional building structures can provide support for the weight of the stacks. As is shown in Figure **4****,** this allows the side-by-side stacks to be positioned on a mezzanine level **29** of the structure as well as the ground floor **30.**

It should be noted that separate versions of the rack **12** are not described in additional details herein for the remaining embodiments because the aspects of the rack illustrated in Figures 1 and 2 can be extended to racks for supporting the containers **11** and lights **13** in the relative positions of the remaining embodiments. It should also be noted that although a preferred embodiment of the rack **12** is illustrated in Figures 1 and 2, various alternative configurations of the rack are possible with different materials, support member arrangements, etc., which will still support the containers **11** and lights **13** in their relative positions. In another example, the rack **12** may be constructed of interconnecting threaded rods with pipe collars to support the containers **11.**

It should also be noted that the relative positions of the lights **13** and the containers **11**, as well as the number of lights and containers, may be modified to suit a particular application. For instance, larger numbers of lights could be used to accelerate growth of the biological material, or larger numbers of containers stacked in a tighter arrangement may be used to grow larger amounts of biological material. Therefore, the combinations of lights and containers are not necessarily restricted to the above-listed configurations and would still fall within the scope of the present invention.

The wall structure **14** of each of the containers **11** is constructed of a light transmissive material which allows the passage of sufficient light to promote growth of the biological material stored in the reservoir **15** defined therein. For instance, the wall structure **14** may be constructed of a glass, such as a borosilicate or flint glass, or a plastic, such as a polycarbonate, polyvinylchloride, polystyrene, TEFLON, silicone, nylon or polyethylene. These materials may be either flexible or relatively rigid. Preferably, the light transmissive material not only allows the passage of some light, but is completely transparent to promote full passage of the light necessary to support growth. However, translucent materials may be used to screen out certain wavelengths or light intensities depending upon such factors as the needs of a biological material or the need to reduce the accumulation of heat in the reservoir **15.**

The term "wall structure" herein refers to any member or collection of members that at least partially defines the reservoir **15.** The wall structure **14** illustrated in the embodiment of Figures 1 and 2 has a cylindrical wall structure with a constant, circular cross-section along its length, which in this case is due to the use of a length of stock piping that is constructed of a transparent material. Preferably, the wall structure has a diameter that ranges from 2 to 12 inches and a length of 10 to 50 feet for growing biological materials of the duckweed family. Such dimensions typically allow 4 to 8 containers **11** to be stacked in a room with conventional ceiling heights. However, it should be recognized that any length or diameter of wall structure may be used as long as a proportionately large media surface can be provided for the growth of biological materials.

Other shapes could also be used for the wall structure **14** including shapes with, and without, a constant cross-section. For instance the wall structure may have a teardrop shape, or some arbitrary or irregular shape constructed to fit lighting needs or available space. Preferably, however, the shape is chosen to maximize the surface area of the portion of a cross-section of the reservoir **15** formed by the wall structure in a plane that is orthogonal to the pull of gravity (i.e., a horizontal plane). For instance, a wall structure **14** having a 6 inch diameter circular, cylindrical cross-section (the embodiment illustrated in Figure 2) and 10 feet of length would have a maximum surface area (at the midpoint between its top and bottom) in the horizontal plane of 35 square feet.

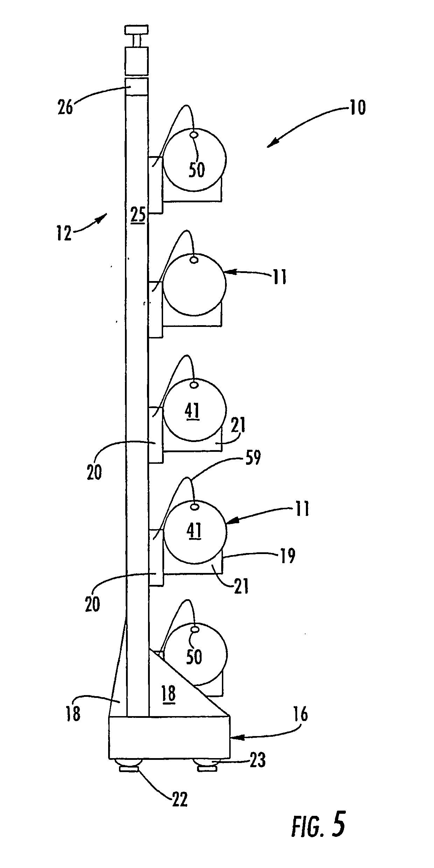

An increase to a 10 inch diameter cross-section of a circular, cylindrical cross-section would result in an increase in media surface area to 42 square feet, as is shown by another embodiment illustrated in Figure 5. However, the tradeoff due to the increase in height of the wall structure **14** is that fewer containers can be stacked within a limited vertical space.

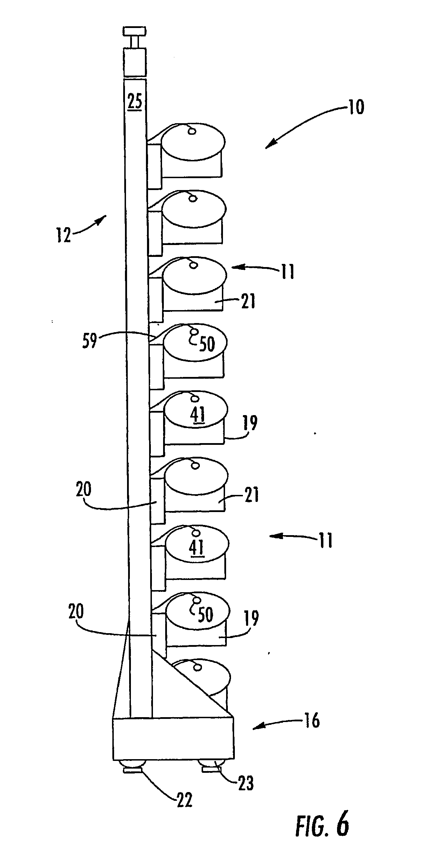

In another embodiment, the cross-section of the wall structure **14** is an oval which has a major axis (i.e., its widest diameter) and a minor axis (i.e., its narrowest diameter), as is shown in Figure 6. Advantageously, the major axis is oriented to be in the horizontal plane so as to maximize the top surface area of the media in the reservoir **15,** while minimizing the height of the wall structure **14** so that more of the containers **11** may be stacked within a fixed vertical space. For instance, the illustrated oval cross-section having major axis of 11.1 inches oriented horizontally and a length of 10 feet the maximum media surface area is 83 square feet. In addition, the relatively short height still allows a large number of the containers **11** per vertical stack.

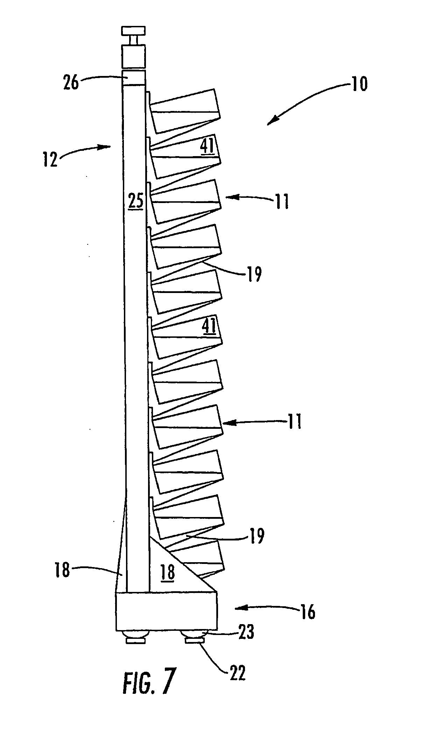

In yet another embodiment, the wall structure **14** has a rectangular cross-section with four corners, as is shown in Figure 7. The horizontal cross-sectional area of the rectangular wall structure **14** is maximized by tilting the container so that two opposing corners are closer to, or in, the horizontal plane and the remaining pair of opposing corners are further away from the horizontal plane. For instance, a corner-to-corner distance of a 10 inch wide rectangular wall structure 10 feet in length results in a media surface area of about 98 square feet. Similar to the oval-shaped wall structure **14,** a relatively large number of the rectangular containers **11** can still be employed in a limited vertical space. Alternatively, the rectangular wall structure **14** could be positioned so that its top and bottom are aligned with the horizontal plane.

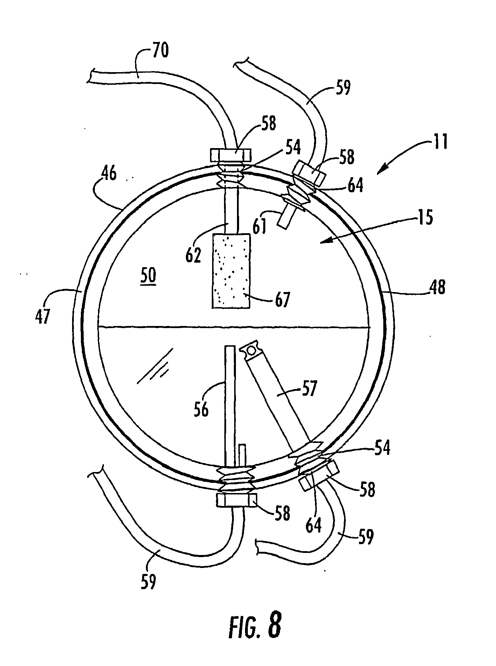

Referring again to Figures 1 and 2, the wall structure **14** has a pair of ends wherein each of the ends is closed off by a clamp **40** and end cap **41** assembly. As is illustrated in more detail in Figure 8, the end cap **41** is a circular plate of transparent material, preferably the same material as used in the wall structure **14,** that is held against one otherwise open end of the wall structure **14.** In addition, the end cap **41** may define one or more openings for access by various devices which will be described in more detail below. Alternatively, the end cap **41** could be constructed of translucent or opaque material if desired, especially if the wall structure **14** is relatively transparent.

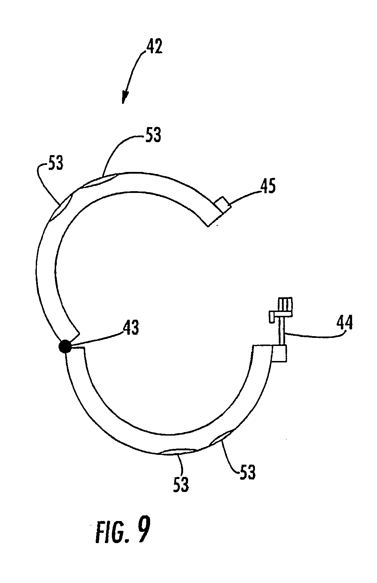

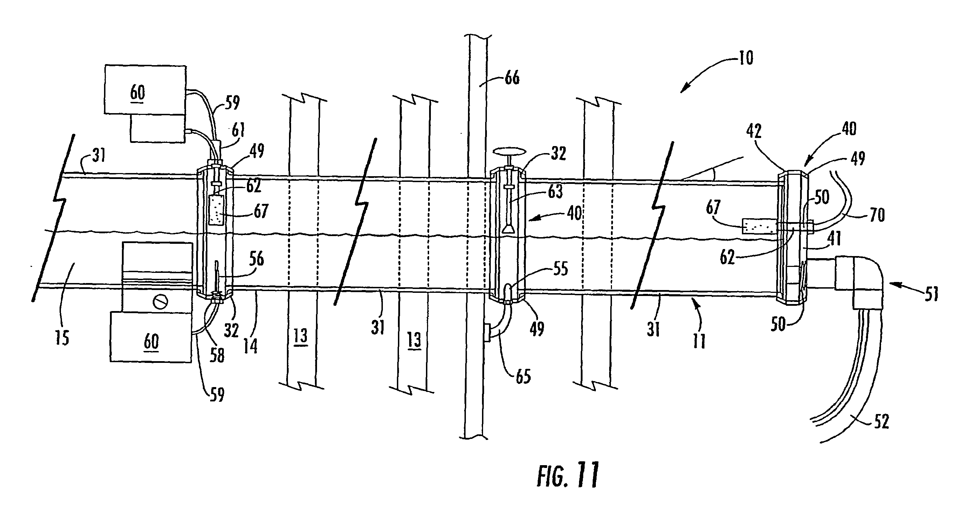

The clamp **40** includes an outer band portion **42** as shown in Figure 9 which is split into two portions connected by a hinge **43.** Opposite the hinge is a locking assembly that includes a screw **44** mounted on one of the portions and a threaded opening defined in a securing flange **45** on the other one of the portions. The outer band portion **42** with both portions connected has a circular shape with an inside diameter corresponding to the outside diameter of the circular cylindrical wall structure. In this manner, the outer band portion **42** can be opened to encircle the wall structure **14** and then secured by tightening the screw **44** in the securing flange **45,** as shown in another embodiment of the present invention illustrated by Figure 11. Of course, the size and shape of the outer band portion can differ so as to match the various sizes and shapes of the wall structures, such as those illustrated in Figures 5, 6 and 7.

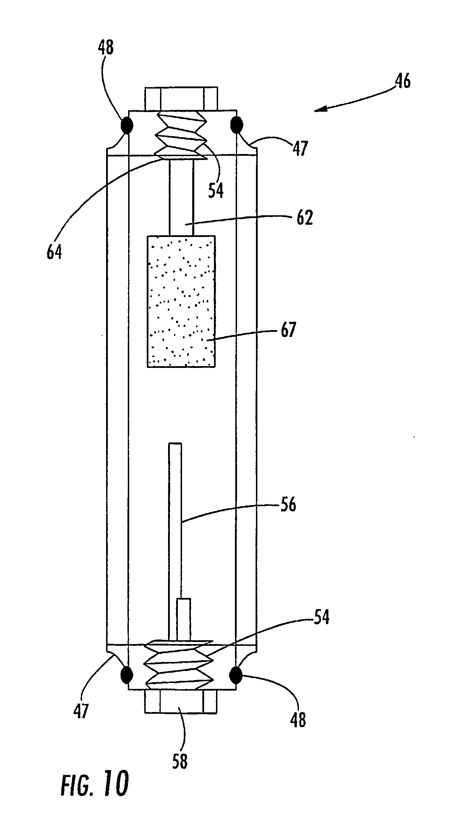

The clamp **40** also includes an inner sealing portion **46,** as shown in Figure 10. The inner sealing portion fitted for the circular, cylindrical wall structure **14** embodiment has the shape of circular, cylindrical ring sized to match the inner diameter of the wall structure, as is shown in Figure 8. In particular, the sealing portion **46** includes a pair of chamfered edges **47** that are spaced across the body of the sealing portion. Each of the chamfered edges extends around the outer periphery of one of the sealing portion's ends and is sized and shaped to receive an end of the wall structure **14** or an edge of the end cap **41.** The sealing portion may be constructed of a metal, such as stainless steel which is corrosion resistant, or an FDA approved composite material, such as acetyl copolymer, which is sufficiently stiff to compress the end cap **41** and wall structure **14** into sealing engagement.

Recessed within each of the chamfered edges **47** is preferably a seal **48,** which in the illustrated embodiment is an O-ring having a circular cross-section. Such positioning of the seal **48** is advantageous because it is interposed and compressed between the end of the wall structure **14** or the edge of the end cap **41** when the outer band portion **42** is tightened thereon, as shown in the separate embodiment of Figure 11. Preferably, the seal is constructed of an inert material, such as silicone, to maintain an aseptic environment in the reservoir **15** and prevent particulate contamination. However, other types of seals could be used that form a seal sufficient to maintain an aseptic environment in the reservoir **15.** For instance, FDA approved elastomer materials such as the aforementioned silicone, polyethylene or rubber could be used for the seals.

Different seal configurations may be employed for different shapes and materials of the wall structure **14,** end cap **41** or clamp **40.** For instance, a gasket-type seal formed of a circular blank defining a hole therethrough could be employed between a wall structure having flat edge defining its end and an end cap that is a flat circular blank held onto the wall structure end using a latch and lever type clamp. In such a case the seal **48** may be constructed of a polymeric or metal material that need not be compressed as much to form a gas and liquid-tight seal due to the increased surface area over which it is applied.

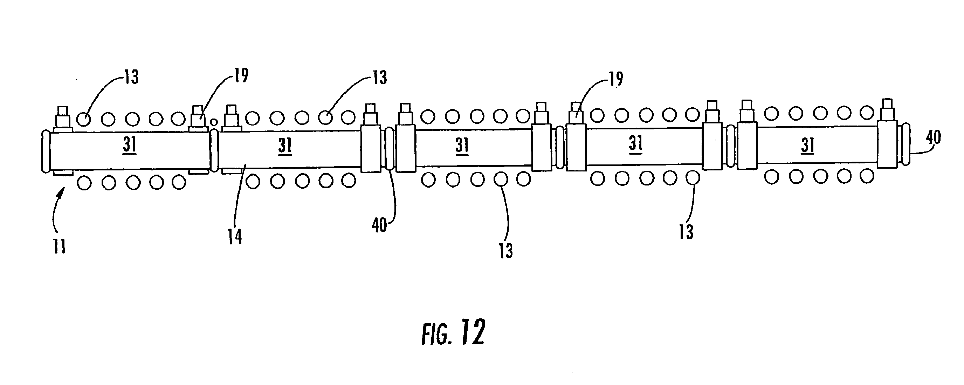

In another embodiment of the present invention, the wall structure **14** of each of the containers **11** is split up into separate portions **31** joined together at adjacent ends by clamps **40,** as shown in Figures 11 and 12. Figure 12 illustrates the use of the separate portions **31** each supported by their own pair of respective cantilevered support members **19.** In addition, the lights **13** extend vertically adjacent the front and back sides of the containers **11.** Of course the multiple-portion container illustrated in Figure 12 could be arranged in a large bank of other containers, as is illustrated by Figure 3, or used in the various other embodiments illustrated and described herein in lieu of the containers having an integral wall structure without sub-portions. The multiple-portion wall structure **14,** however, has the advantage of easy disassembly for compact transport and modifiability of the length of the containers **11.**

As is shown in Figure 11, one of the wall structure portions **31** has one end joined to an adjacent wall structure portion and is closed off by the end cap **41** at the other end. In particular, the end cap is held to the end of the wall structure portion by the clamp **40.** Facilitating clamping attachment is a flared edge **32** that extend outwards in a radial direction at the ends of each of the wall structure portions **31.** Preferably, the flared edge is rounded so as to fit snugly within the chamfered edges **47** of the clamp **40.** In addition, the band portion **42** of each clamp **40** may have a pair of inwardly directed flanges **49** spaced apart on opposite ends of the band portion.

During assembly, the flared edge of the wall structure portion **31** is seated against the seal **48** and its respective chamfered edge of the sealing portion **46.** Then, the inwardly directed flange at one end of the band portion **42** is extended over the outwardly directed flared edge **32** of the wall structure portion. As the band portion **42** is tightened, the inwardly directed flange **49** extends downwards onto the outer surface of the wall structure **14** at the base of the flared edge **32.** This holds the flared edge by forming an inner diameter smaller than the diameter of the flared edge, thereby restraining the wall structure portion against axial movement.

In order to facilitate providing an aseptic environment while still allowing control of the environment within the reservoir **15,** a plurality of threaded openings are defined through the inner band portion **42** and/or the end cap **41** to allow passage therethrough of various sampling, measurement and supply devices. Referring to Figure 11, threaded openings **50** through the end cap allow the passage of main fill and drain nozzles **51.** Each of these nozzles can receive or supply media and biological materials at a relatively high rate and therefore have relatively large diameters.

In addition, the nozzles **51** are attached to elbows and main fill and drain conduits **52** that extend downwards from the end cap **41.** As is shown in Figures 1 and 2, the main fill and drain conduits **52** are preferably individualized to each of the containers **11** so that there is no cross-talk between the biological materials produced in one container with another container. Also, individualization isolates incidence of cross-contamination and allows the use of customized media for each of the containers. The nozzles **51** further provide the ability for clean-in-place (CIP) to pharmaceutical standards. Upstream, the fill conduits **52** may connect to a central header manifold from which the conduits branch, each having one or more valves to control flow.

As another option, the bioreactor assembly **10** may further include a disposable container liner that is configured to extend around the inside of each of the containers **11.** For instance, such a liner could be inflatable to overly the portions of the container defining the reservoir **15** and translucent to allow light therethrough, or could be a translucent polymeric sleeve that slides into the wall structure **14.** Advantageously, the container liner would promote cleaning procedures by being removable and disposable, allowing insertion of another container liner. Cleaning may also be facilitated through use of a special end cap **41** having particularly large openings defined therethrough for connection of large media extraction nozzles. Attachment of the end cap facilitates drawing of a vacuum in the reservoir **15** during media extraction.

Beyond primary supply and removal of the media, the media can be sampled and measured by other devices. For instance, openings **53** defined in the band portion **42** and threaded openings **54** defined in the sealing portion **46** of each clamp **40** can allow insertion and securing of a sampling nozzle **55,** a multiple-level temperature probe **56** and a pH probe **57,** as is shown in Figures 8, 10 and 11. As shown most clearly in Figure 8 (wherein the band portion **42** of the clamp **40** is not shown for additional clarity), each of the probes can include a bolt head **58** attached at the end of a threaded portion **64** which allows the threaded portion to be advanced into the threaded opening **54** to secure it to the clamp **40.** The sampling drain is attached similarly, as shown in Figure 11. Although illustrated with the threads extending into the reservoir **15,** the portion of the wall structure **14** most adjacent to the reservoir could define a relatively smooth cylindrical opening having a series of O-rings or other seals extending along its length to protect against leakage.

Extending from the threaded portion into the media is the probe (or nozzle) itself. For the probes, extending from the bolt head **58** are electrical leads **59** that connect to a conventional electronic measurement and control system **60.** The sampling nozzle **55** has its own conduit **65** connected to a sampling and supply network **66.** In this manner, the media can be measured for pH level, measured for temperature (at multiple levels in the media) or sampled for other measurements without opening of the containers **11** and the risk of contamination. Preferably, all of the threaded openings described herein are sealed against passage of contaminants, such as through use of polymeric tape, solder, a washer and seal combination, etc. As another alternative, once the threaded portion is secured within the threaded opening they could be welded, glued or otherwise permanently attached for a tight seal. Advantageously, for such permanent attachments threads may not even be required as long as the devices can be appropriately positioned during the welding or attachment process.

In addition to the supply, removal and measurement of media, air or other gasses in the reservoir **15** can be supplied, removed and measured using various other devices attached in a similar manner to the above-described devices. For example, additional threaded openings **50, 54** allow passage of an air temperature probe **61,** a gas supply nozzle **62** and a gas exit nozzle **63.** Each of these devices is secured in its respective threaded opening with its own bolt head **58** and threaded portion **64.** The air temperature probe **61** allows the air temperature to be measured. Gas supply and exit nozzles **62, 63** allow control of the type, temperature, flow rate and other characteristics of the gasses in the reservoir **15.** Preferably, the gas exit nozzle **63** is biased so as to allow flow in only a single direction, thereby preventing the infiltration of contaminants. At one end of the gas supply nozzle **62** is preferably a sparger **67** that diffuses the air supply so that it does not unduly disturb the media and biological matter within the reservoir **15.** At the other end of the gas supply nozzle is a gas supply line **70.**

It should be noted that other measurements within the reservoir **15** could also be made with a variation of other devices depending upon the information desired by the user. For instance, a gas composition probe could be used to sample the amount of CO₂ which would be used as feedback to modify the composition of gasses being added or removed by the nozzles **62, 63.** The measurement, supply and removal devices discussed herein may also have different sizes, configurations and placements depending upon the desired frequency, accuracy, speed and other qualities of their performance.

In addition, the devices could also be extended through the container at other locations and portions of the containers **11** depending upon various needs of the user such as accessibility, tolerability of the container materials to openings, the risk of leakage and contamination, etc. For instance, the devices could extend through openings defined in the wall structure **14** or other components of the bioreactor assembly **10,** in addition to the end cap **41** of the clamps **40.** As another alternative a short section of the wall structure **14** (e.g., 4 to 6 inches in length) could define the openings for various devices. For instance, the nozzles **62, 63** could be supported and extend through the short section of wall structure and the short section of wall structure could be secured to the remaining wall structure with a victualic-type clamp. The short section could be removed and reattached for easy maintenance and cleaning.

The gas supply and removal nozzles **62, 63** could also be employed with a pump having sufficient power to reduce the gas pressure within the reservoir **15** prior to harvest of the biological materials in order to increase protein production by the biological materials. Alternatively, during a growth phase the gas pressure can be increased to promote growth of the biological materials in the reservoir. Notably, this is particularly effective for the media surface-borne plants which have large portions exposed to the gasses in the reservoir. Also, the air-tight construction of the reservoir of the present invention facilitates manipulation of the gas pressure therein.

It should be further recognized that although the illustrated clamp **40** is preferred for the illustrated wall structure **14** configurations, other types of clamps may also be employed herein to connect different portions of the bioreactor assembly **10.** Alternative clamp configurations can address various factors, as desired, such as easy application and removal, firm attachment (which would benefit from the above-mentioned lever-type variation or a lever and ratchet design that allows progressive tightening), a complementary seal design that ensures an air and liquid-tight seal to ensure an aseptic environment within the reservoir **15,** corrosion resistance, biocompatibility, use of acceptable materials under FDA regulations for pharmacological manufacturing processes and ability to support various measurement and sampling devices while maintaining the aseptic reservoir environment.

It should also be noted that although the above-described embodiments each has a continuous reservoir **15** extending in along a single major axis (i.e., a length extending in its longest dimension) and having a constant cross-section, the present invention should not be limited to such shapes. The wall structure **14,** end cap **41** and other portions of the containers **11** defining the reservoir 15 can have several twists, turns, bifurcations and deviations as long as the media within the reservoir can be filled to a level defining a relative large media surface area for the support of surface-borne biological materials, such as duckweed plants.

Generally, this reservoir will have one or more major portions that each have a primary axis wherein the axes of all or most of the portions share a common plane. In this manner, the reservoir can be oriented (by orienting the container) until it is substantially horizontal (i.e., orthogonal to the pull of gravity) so that the flowable media forms the relatively large surface area. The term "substantially horizontal" is used herein because some angle in the major axis or axes may be desired to induce flow for processing purposes. For instance, the containers 11 illustrated in Figure 1 have about a one inch drop per 50 feet in the direction of the main fill and drain nozzles 51 to facilitate fill and drain operations. A steeper drop could be used to further urge the media in the drop direction, but preferably the drop does not cause one end of the container to fill with media, or the media to fill to a height wherein the wall structure **14** prohibits full upwards growth of biological material on the media surface. Therefore, longer reservoir lengths 15 will typically require a less steep drop unless the reservoir is relatively tall compared to its length.

During initial use, the containers **11** are filled with the media using the main fill and drain nozzles **51** to supply relatively large volumes of the media. Biological materials can also be added using the main nozzles **51,** or may be added when initially assembling the containers **11.** Preferably, a surface-borne biological material is added such as plants from the duckweed family, or the aquatic plant species described above, that require light to proliferate via photosynthesis. As the reservoir **15** is filled it is monitored either visually, or automatically, to determine at which point the media reaches a level at which a maximized surface area is defined. In the case of the embodiment illustrated in Figures 1 and 2, this is at approximately the half-full point.

After the biological material and media are added, the power is supplied to the lights **13** (or the lights may have already been on) so as to cast light through the transparent wall structure **14** into the reservoir **15.** Over time, the biological materials draw energy from the light and nutrients from the media and begin to proliferate. In the case of biological materials used for pharmacological purposes, the biological materials begin to secrete peptides and proteins into the surrounding media.

Also during this time, the various probes **56, 57, 61** are used to measure the properties (temperature, pH, CO₂ composition, etc.) of the gaseous and media environment in the reservoir. In turn, this data is collected and used to control the intensity of the lights **13,** the temperature and convection properties of the ambient air around the containers **11,** the temperature and amounts of gasses and media supplied to the reservoir **15** through the gas supply nozzle **62** and fill and drain conduits **52.** In addition, the sampling nozzle **55** can be used to take small samples to determine the progress of the secretions. Such progress may also be used to determine the various aforementioned conditions within the reservoir **15.**

At a certain point, such as when the media is exhausted or a complete harvesting of the biological materials is desired, the entire contents of the reservoir **15** can be flushed out of the main fill and drain nozzles **51** and conduits **52.** After such flushing, cleaning compounds can be run through the system using the same nozzles and conduits. Alternatively, some type of steady state can be established wherein the expressed products of the biological materials can be continuous sampled, or partially drained, and the media and gasses refreshed, so that the growth and expression process is continues almost indefinitely.

The present invention has many advantages. Overall, the bioreactor assembly **10** allows the production of clinical and commercial scale quantities of biopharmaceuticals from genetically modified plants in a contained, aseptic environment. For example, the use of containers **11** defining reservoirs **15** for partial filling with media provides a relatively large surface for the large-scale production of surface-borne biological materials, such as duckweed plants. In addition, use of the clamps **40** having seals to interconnect the various portions of the container wall structure **14** and sealed openings **50, 54** for insertion of various measurement and supply devices ensures a clean and aseptic environment to promote the growth of the biological materials for medical uses. The clamping system also allows for easy assembly and disassembly of the containers **11** for maintenance and modification. The measurement and supply devices ensure that the environment within the reservoir **15** is tightly controlled to maximize growth and expression of the biological materials therein.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. An assembly for holding a media and supporting growth of a biological material requiring light for proliferation, said assembly comprising:
at least one light source; and
at least one container positioned adjacent the light source, said container having a flexible light transmissive wall structure defining an elongate, aseptic reservoir, said elongate reservoir having a major axis with a substantially horizontal orientation wherein said reservoir is capable of being partially filled with media so as to create a media surface on which the biological material is supported.

2. An assembly according to Claim 1, wherein the wall structure has a constant cross-section along said major axis.

3. An assembly according to Claim 2, wherein the cross-section is a rectangular cross-section.

4. An assembly according to Claim 1, wherein the wall structure is between 4 and 20 feet in length.

5. An assembly according to Claim 1, additionally including a rack, with a plurality of said containers supported by said rack and arranged in a vertical stack with spacing therebetween.

6. An assembly according to Claim 5, wherein the light source includes a plurality of electrically powered lights positioned in the spacing between the plurality of said containers, the plurality of electrically powered lights extending in the substantially horizontal plane.

7. An assembly according to Claim 1, wherein the wall structure has a constant oval-shaped cross-section along said major axis and wherein the oval shaped cross-section has a major axis extending in the substantially horizontal plane.

8. An assembly according to Claim 1, further comprising at least one of a gas supply nozzle, a gas exit nozzle, an air temperature probe, a pH probe, a sampling drain, a gas composition probe and a media temperature probe extending into the reservoir through an opening defined by the container.

9. An assembly for holding a media and supporting growth of a biological material requiring light for proliferation, said assembly comprising:
a support rack;
a plurality of elongate laterally extending containers carried by said support rack and arranged in a stack spaced apart vertically from one another, each container having a flexible light transmissive wall structure defining an elongate, aseptic reservoir, said elongate reservoir having a major axis with a substantially horizontal orientation wherein the reservoir is capable of being partially filed with media so as to create a media surface on which the biological material is supported; and
at least one light source carried by said support rack and mounted adjacent to said containers for illuminating the containers.

10. An assembly according to Claim 9, wherein said support rack includes a plurality of upright support members, and upper and lower laterally extending support rails interconnecting said upright support members, and wherein said containers are mounted to said upright support members.

11. An assembly according to Claim 9, wherein said at least one light source comprises a plurality of elongate fluorescent tubes mounted to said rack.

12. An assembly according to Claim 11, wherein the tubes extend vertically and are laterally spaced from one another.

13. An assembly according to Claim 11, wherein the tubes extend substantially horizontally and generally parallel to the containers.

14. A method of growing in a liquid media a biological material requiring light for proliferation, said method comprising:
providing at least one flexible light transmissive container defining a reservoir having a major axis with a substantially horizontal orientation;
filling the reservoir with the liquid media until a partial fill level is reached so as to define a top surface of the media extending along a length of the reservoir;
adding the biological material to the reservoir and supporting the biological material on the top surface of the media; and
exposing the container to a light source so as to promote growth of the biological material via photosynthesis.

15. A method of Claim 14, further comprising sealing the reservoir against contamination after filling the reservoir with liquid media and adding the biological material.

16. A method of Claim 14, wherein providing the light transmissive container includes clamping portions of the light transmissive container together using one or more clamps.

17. A method of Claim 14, further comprising accessing the reservoir through an opening defined in the container.

18. A method of Claim 17, wherein accessing the reservoir includes inserting a gas supply nozzle through the clamp opening and supplying gas to the reservoir.

19. A method of Claim 17, wherein accessing the reservoir includes inserting a temperature probe through the clamp opening and measuring a temperature within the reservoir.

20. A method of Claim 17, wherein accessing the reservoir includes inserting a pH probe through the clamp opening and measuring a pH of the media within the reservoir.

21. A method of Claim 17, wherein accessing the reservoir includes draining a sample through the clamp opening.

22. A method of Claim 14, wherein filling the reservoir includes supplying the media through an opening defined in an end of the container.

23. A method of Claim 14, further comprising draining the liquid media from the reservoir after filling the reservoir.

24. A method of Claim 14, further comprising automatically measuring and controlling one of a temperature, a media pH, the media fill level, gas pressure and gas concentration.

25. A method of Claim 14, further comprising supplying conditioned air around the container to control a temperate within the reservoir.

26. A method of Claim 14, further comprising heating and circulating the media.

27. A method of Claim 14, further comprising mounting a plurality of the containers in a stack spaced apart vertically from one another.
